# EUROPÄISCHE PATENTSCHRIFT

(11) **EP 2 134 286 B1**
(45) Veröffentlichungstag und Bekanntmachung des Hinweises auf die Patenterteilung: **21.11.2018**
(21) Anmeldenummer: 08716525.4
(22) Anmeldetag: 13.03.2008
(51) Int. Cl.: A61C 1/00

(54) **ANORDNUNG ZUM STEUERN MEDIZINISCHER GERÄTE, INSBESONDERE EINES ZAHNÄRZTLICHEN ARBEITSPLATZES**
ARRANGEMENT FOR CONTROLLING MEDICINAL DEVICES, IN PARTICULAR OF A DENTAL WORK STATION
SYSTÈME DE COMMANDE D'APPAREILS MÉDICAUX, NOTAMMENT D'UN POSTE DE TRAVAIL DENTAIRE

(30) Priorität: 20.03.2007 DE 102007013310
(43) Veröffentlichungstag der Anmeldung: 23.12.2009
(73) Patentinhaber: Kaltenbach & Voigt GmbH, 88400 Biberach (DE)
(72) Erfinder: STEHLIK, Birgit, 91091 Grossenseebach (DE); GMEINDER, Hermann, 88400 Biberach (DE); SCHÖNENBERGER, Richard, 88299 Leutkirchen (DE); KECK, Franz, 88400 Biberach (DE)
(74) Vertreter: Thun, Clemens
(86) Internationale Anmeldenummer: PCT/EP2008/002028
(87) Internationale Veröffentlichungsnummer: WO 2008/113513

(56) Entgegenhaltungen:
- EP-A- 0 891 745
- EP-A1- 1 714 617
- WO-A-2005/053561
- DE-A1- 2 231 265
- DE-A1- 3 302 558
- FR-A2- 2 502 936

## Beschreibung

Die vorliegende Erfindung betrifft eine Anordnung gemäß Anspruch 1, welche zum Ansteuern medizinischer Geräte, insbesondere eines zahnärztlichen Arbeitsplatzes vorgesehen ist. Insbesondere betrifft die vorliegende Erfindung einen sog. zahnärztlichen Fußanlasser.

Zahnärztliche Arbeits- oder Behandlungsplätze sind üblicherweise ortsfest in einem Praxisraum installiert und weisen eine Mehrzahl von zahnärztlichen Untersuchungs- und Behandlungsinstrumenten auf, welche von einer Zentraleinheit mit Energie und/oder Behandlungsmedien versorgt werden. Auch eine Ansteuerung der verschiedenen Instrumente erfolgt über die Zentraleinheit. Ein derartiger Arbeitsplatz enthält in der Regel einen Kompressor zur Erzeugung von Druckluft, welche insbesondere zum Antrieb von turbinengetriebenen Bohrinstrumenten genutzt wird.

Da ein Zahnarzt während der Behandlung sich in der erster Linie auf die Handhabung der Behandlungsinstrumente konzentrieren sollte, sollte ein Ansteuerung der verschiedenen Instrumente, bspw. eine Drehzahlverstellung oder dgl. in möglichst einfacher Weise durchgeführt werden können. Als geeignete Bedienvorrichtung hierfür hat sich in der Vergangenheit ein Gerät durchgesetzt, welches in der Regel als Fußanlasser bezeichnet wird und entsprechend der Bezeichnung mit dem Fuß zu betätigen ist. Während in der Vergangenheit üblicherweise Fußanlasser lediglich eine Veränderung der Drehzahl mit Hilfe eines Pedals oder Schwenkhebels ermöglichten, weisen Fußanlasser der neueren Generation oftmals mehrere Bedienelemente auf, über die umfangreich verschiedenste Funktionen eines zahnärztlichen Arbeitsplatzes aktiviert bzw. hinsichtlich ihrer Betriebsparameter verändert werden können. So besteht bspw. nunmehr auch die Möglichkeit, in einfacher Weise die Position und Steuerung des Patientenstuhls zu vorzunehmen oder andere Funktionen zu aktivieren. Nachdem es ursprünglich üblich war, den Fußanlasser über ein Steuerkabel mit der Zentraleinheit des zahnärztlichen Arbeitsplatzes zu verbinden, wurden in der Vergangenheit auch vereinzelt Lösungen beschrieben, eine drahtlose Übermittlung der Steuersignale vorzunehmen. Derartige Lösungen, wie sie bspw. aus der

DE 33 16 710 A1 oder der WO 2004/019751 A1 bekannt sind, bringen den Vorteil mit sich, dass kein separates Kabel zwischen dem Fußanlasser und dem zahnärztlichen Arbeitsplatz verlegt werden muss. Da ein derartiges am Boden verlaufendes Kabel immer auch die Gefahr des Stolperns mit sich bringt, kann durch die drahtlose Ansteuerung ein erhöhter Komfort erzielt werden.

Aus der EP 0 891 745 A2 ist eine medizinische Vorrichtung bekannt, bei der Steuersignale, die mithilfe eines Fußschalters erzeugt werden, via Funk zu einem Hochfrequenz-Chirurgiegerät übermittelt werden. Aus der Schrift WO 2005/053561 A2 ist eine Anordnung mit einem Fußschalter für die Bedienung eines dentalen Handgeräts bekannt, wobei ebenfalls eine Funkübertragung von Steuersignalen vorgesehen ist.

Aus der EP 1 714 617 A1 ist eine Vorrichtung zum Ansteuern medizinischer Geräte bekannt, die ein Fuß-Bedienung mit einem horizontal schwenkbaren Fersen-Element aufweist. Mit Hilfe der Fuß-Bedienung lassen sich Steuersignale erzeugen und drahtlos an ein medizinisches Gerät übermitteln.

Aus der FR 2 502 936 A2 ist ein zahnärztlicher Behandlungsplatz mit einem Fußanlasser bekannt. Der Fußanlasser umfasst eine Fußraste, die sich entgegen der Wirkung von Federn aus einer Mittellage heraus seitlich auslenken lässt. Auch aus der DE 2 231 265 A1 ist eine insoweit vergleichbare Vorrichtung bekannt.

Basierend auf diesen neueren drahtlosen Systemen zur Ansteuerung eines zahnärztlichen Arbeitsplatzes liegt der vorliegenden Erfindung nunmehr die Aufgabe zugrunde, derartige Vorrichtungen weiter zu verbessern, um die Betriebseigenschaften zusätzlich zu optimieren und eine angenehme Handhabung für den Zahnarzt sicherzustellen.

Die Aufgabe wird durch die in den unabhängigen Ansprüchen definierte Erfindung gelöst. Vorteilhafte Weiterbildungen der Erfindung sind Gegenstand der abhängigen Ansprüche.

Ein erster Aspekt der vorliegenden Erfindung betrifft dabei die Ausgestaltung der Bedienvorrichtung im Hinblick auf das Pedal, welches bspw. zur Drehzahleinstellung genutzt werden kann. Hierbei ist erfindungsgemäß vorgesehen, dass Mittel vorgesehen sind, über welche dieses schwenkbare Eingabeelement nach einem Verschwenken aus einer Ausgangs- bzw. Ruheposition heraus automatisch wieder in diese Ausgangsposition zurückgeführt wird, sobald es losgelassen wird. Erfindungsgemäß wird dementsprechend eine sog. Mittenzentrierung für das Eingabeelement geschaffen, welche sicherstellt, dass dieses nach Loslassen jederzeit wieder in seine Ausgangsposition zurückkehrt. Derartige Mittenzentrierungen waren bereits aus dem Stand der Technik, bspw. der DE 33 02 558 A1 bekannt, allerdings nur bei solchen Fußanlassern, welche über eine Kabelverbindung mit dem zahnärztlichen Arbeitsplatz verbunden waren. Grund hierfür ist, dass die Rückstellung des Anlasserpedals mit Hilfe der von dem zahnärztlichen Arbeitsplatz zur Verfügung gestellten Druckluft erfolgte. Gemäß der vorliegenden Erfindung wird nunmehr erstmalig vorgeschlagen, eine derartige Mittenzentrierung auch bei Fußanlassern vorzusehen, welche von dem Arbeitsplatz getrennt sind und bei denen dementsprechend keine Druckluft zur Rücksteuerung des Pedals genutzt werden kann. Spezielle Maßnahmen zur Realisierung dieser Mittel, über welche die Rückführung des Pedals erzielt wird, sind Gegenstand der abhängigen Ansprüche.

Gemäß diesem ersten Aspekt der vorliegenden Erfindung wird also eine Anordnung zum Ansteuern medizinischer Geräte, insbesondere eines zahnärztlichen Arbeitsplatzes vorgeschlagen, welche eine Bedienvorrichtung zum Generieren von Steuer- und/oder Stellsignalen für das medizinische Gerät, eine der Bedienvorrichtung zugeordnete Sendeeinrichtung zur drahtlosen Übermittlung der Steuer- und/oder Stellsignale sowie eine dem medizinischen Gerät zugeordnete Empfangseinheit zum Empfangen der Steuer- und/oder Stellsignale sowie zur Weiterleitung dieser an das medizinische Gerät aufweist, wobei die Bedienvorrichtung ein mit einem Fuß zu betätigendes Eingabeelement aufweist, welches schwenkbar gelagert ist, und wobei erfindungsgemäß Mittel vorgesehen sind, über welche das schwenkbare Eingabeelement nach einem Verschwenken aus einer Ausgangsposition automatisch wieder in diese Ausgangsposition zurückgeführt wird. Das schwenkbare Eingabeelement ist dabei in einer horizontalen Ebene veschwenkbar.

Durch die erfindungsgemäße Rückführung bzw. Mittenzentrierung des Fußanlasser-Pedals wird dem Zahnarzt ein angenehmeres Bedienen des zahnärztlichen Arbeitsplatzes ermöglicht. Wird nämlich bspw. das Pedal wieder losgelassen, so bringt die Rückführung den Effekt mit sich, dass gleichzeitig auch die entsprechende Drehzahl des aktuell angesteuerten Handstücks wieder auf Null zurückfährt. Hierbei ist kein umständliches manuelles Zurückfahrens des Pedals erforderlich, was die Konzentration des Zahnarztes beanspruchen würde. Weiterhin erfindungsgemäß ist allerdings vorgesehen, dass auf Wunsch die automatische Rückführung auch deaktiviert wird. In diesem Fall würde das Pedal dann in der entsprechend verschwenkten Stellung verharren, nachdem es losgelassen wurde.

Nachfolgend soll die Erfindung anhand der beiliegenden Zeichnungen näher erläutert werden. Es zeigen:
- Fig. 1: in Gesamtansicht einen zahnärztlichen Behandlungsplatz, der durch eine erfindungsgemäße drahtlose Bedienvorrichtung angesteuert werden kann;
- Fig. 2: den drahtlosen Fußanlasser zur Steuerung des Arbeitsplatzes in vergrößerter Ansicht;
- Fig. 3: eine schematische Darstellung der Kommunikation zwischen zahnärztlichem Behandlungsplatz und Fußanlasser;
- Fig. 4: ein Schema zur Verdeutlichung des Energiemanagements des erfindungsgemäßen Fußanlassers und
- Fig. 5 - 8: Darstellungen der Mittel zur Mittenzentrierung des Anlasser-Pedals.

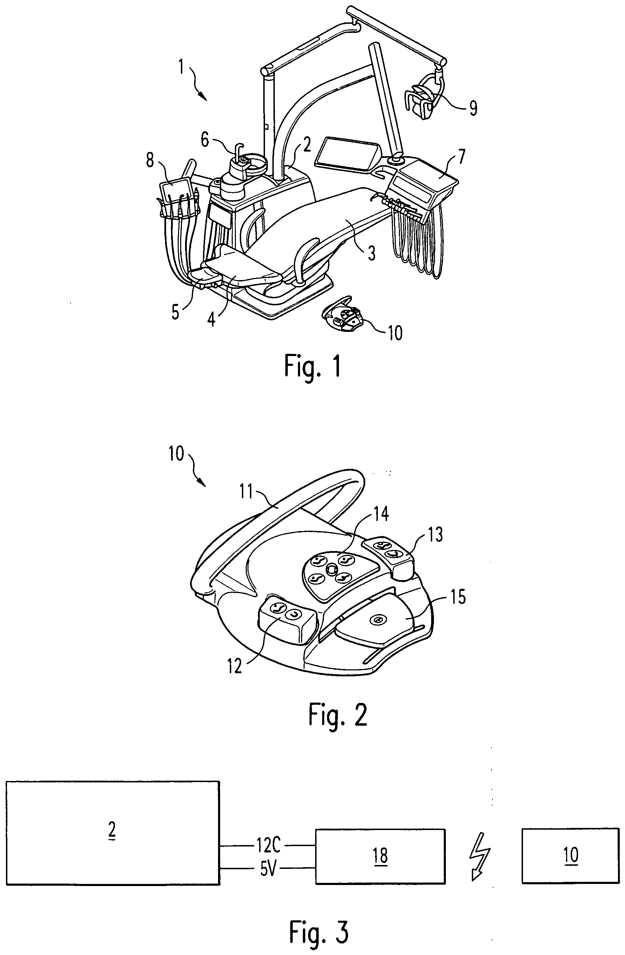

Fig. 1 zeigt die Ansicht eines allgemein mit dem Bezugszeichen 1 versehenen zahnärztlichen Arbeitsplatzes. Zentrales Element des Arbeitsplatzes 1 ist eine säulenförmige Zentraleinheit 2, welche der Energieversorgung und Ansteuerung der verschiedenen Komponenten des Arbeitsplatzes 1 dient. Auch die Zufuhr von Medien wie Luft und/oder Wasser erfolgt über diese Zentraleinheit 2, an der die unterschiedlichsten Einheiten des Arbeitsplatzes 1 befestigt bzw. angeordnet sind. Hierbei handelt es sich bspw. um den Patientenstuhl 3 mit der verschwenkbaren Rückenlehne 4 und einer ggf. in Längsrichtung verstellbaren Kopfstütze 5, um ein sog. Patiententeil 6, ein Arztelement 7, ein Helferinnenelement 8 sowie um eine Behandlungsleuchte 9. An dem Patiententeil 6 befindet sich eine Speischale sowie eine Versorgungseinrichtung, über welche Wasser zum Spülen des Patientenmundraums zur Verfügung steht. Das Arztelement 7 sowie das Helferinnenelement 8 wiederum weisen Instrumentenablagen auf, die zur Halterung unterschiedlicher Untersuchungs-und/oder Behandlungsinstrumente dienen. Je nachdem, welches Instrument gerade aus der Ablage entnommen wurde, wird dieses in geeigneter Weise von der Zentraleinheit 2 mit Strom, Druckluft sowie Luft und/oder Wasser versorgt, und zwar abhängig davon, um welches Instrument es sich handelt und welche Antriebseinheit (Luftturbine oder Elektromotor) verwendet wird.

Die Ansteuerung bzw. Aktivierung der zuvor beschriebenen verschiedenen Komponenten des zahnärztlichen Arbeitsplatzes 1 durch den Zahnarzt erfolgt mit Hilfe eines sog. Fußanlassers 10, der vergrößert in Fig. 2 dargestellt ist. An diesem Fußanlasser 10, der in der Nähe des Arbeitsplatzes 1 auf dem Boden zu positionieren ist, sind mehrere Bedienelemente vorgesehen, über die unterschiedliche Funktionen des Arbeitsplatzes 1 aufgerufen bzw. aktiviert werden können. Hierbei handelt es sich einerseits um einen Bügelschalter 11, zwei seitliche Fußtasten 12 und 13 zur Steuerung unterschiedlicher Behandlungsmedien, einen Kreuztaster 14 zur Verstellung des Patientenstuhls 3 sowie zur Beeinflussung der Motordrehrichtung sowie um ein Fußpedal 15. Das Fußpedal 15 als wesentliches Eingabeelement ist in einer Ebene, welche parallel zur Bodenfläche verläuft, um eine innerhalb des Gehäuses 16 des Fußanlassers 10 befindliche Aufnahme verschwenkbar und dient bspw. dazu, das aktuell aus der Instrumentenablage des Arztelements 7 entnommene Instrument anzusteuern. Durch ein horizontales Verschwenken des Fußpedals 15 kann bspw. dessen Drehzahl eingestellt werden.

Grundsätzlich gesehen sind derartige Fußanlasser, wie sie in Fig. 2 dargestellt sind, bereits bekannt. Eine erste Besonderheit besteht nunmehr darin, dass die Kommunikation zwischen Fußanlasser 10 und Zentraleinheit 2 des zahnärztlichen Behandlungsplatzes 1 drahtlos erfolgt. Hierzu weist der Fußanlasser 10 intern eine Sendeinheit auf, welche zur drahtlosen Übermittlung der durch die Betätigung der verschiedenen Bedienelemente generierten Steuer- und/oder Stellsignale ausgebildet ist. Ferner ist der Zentraleinrichtung 2 des Behandlungsplatzes 1 eine Empfangseinheit 18 zugeordnet, welche dem Empfang der Stell- und/oder Steuersignale sowie zur Weiterleitung dieser Signale an die Zentraleinheit 2 dient. Durch den Wegfall des bislang verwendeten relativ starren Kabels zwischen Fußanlasser 10 und Behandlungseinheit wird die Einschränkung der Bewegungsfreiheit für den Zahnarzt aufgehoben. Ferner ist kein Kabel mehr vorhanden, welches als Stolperfalle dienen kann.

Die Energieversorgung des der Behandlungseinheit 2 zugeordneten Empfängers 18 erfolgt durch die Behandlungseinheit 2 selbst. Hierzu ist entsprechend der Darstellung von Fig. 3 eine 5-Volt-Leitung vorgesehen, über welche eine erste Verbindung zwischen beiden Komponenten erfolgt. Der Fußanlasser 10 wiederum weist intern einen Akkumulator auf, der in regelmäßigen Abständen aufgeladen werden muss, um eine ausreichende Energieversorgung sicherzustellen.

Die Kommunikation zwischen Fußanlasser 10 und Empfänger 18 erfolgt vorzugsweise über Funk, es könnten allerdings auch andere Technologien zur drahtlosen Kommunikation (bspw. Infrarot oder dgl.) eingesetzt werden. Die Weiterleitung dieser Signale zwischen Empfänger 18 und Zentraleinheit 2 wiederum erfolgt über eine I2C-Schnittstelle, wofür eine weitere Leitung zwischen Zentraleinheit 2 und Empfänger 18 verlegt ist. Es handelt sich hierbei um die gleiche Schnittstelle, die auch normalerweise zur Übermittelung der Steuersignale bei kabelgebundenen Fußanlassem verwendet wird. Damit bestünde die Möglichkeit, herkömmliche Fußanlasser in einfacher Weise durch die dargestellte Kombination bestehend aus Funk-Fußanlasser 10 und Empfänger 18 zu ersetzen, wobei allerdings beim dargestellten Ausführungsbeispiel der Empfänger 18 vorzugsweise in die Zentraleinheit 2 integriert ist.

Vorzugsweise erfolgt eine zyklische Übertragung der Daten via Funk von dem Sender des Fußanlassers 10 zu dem Empfänger 18, und zwar sowohl im Betrieb als auch im Ruhezustand, um eine Synchronität zwischen Fußanlasser 18 und zentraler Einheit 2 sicherzustellen. Hierbei ist ferner zur Inbetriebnahme des Systems ein Konfigurations-Algorithmus implementiert, der sicherstellt, dass der Empfänger 18 sowie der Sender des Fußanlassers 10 exklusiv miteinander verbunden werden. Über verschiedene Selektierungsgedanken- und Differenzierungsschritte erfolgt hierbei eine individuelle Adressvergebung zwischen Sender und Empfänger, um Fremdbedienungen auszuschließen. Ferner ist auf diese Weise sichergestellt, dass mehrere derartiger Funk-Fußanlasser in einer größeren zahnärztlichen Praxis betrieben werden können.

Da beim Betrieb des Fußanlassers 10 nicht der Fall auftreten darf, dass es aufgrund einer plötzlichen Unterbrechung der Funkverbindung mangels ausreichender Energieversorgung des Fußanlassers 10 zu unkontrollierbaren Zuständen kommt, sind erfindungsgemäß besondere Sicherungsmaßnahmen vorgesehen, welche derartige Fälle verhindern sollen. So ist zunächst einmal eine Überwachung des Ladezustands des Akkumulators vorgesehen, wobei mittels optischer und akustischer Anzeigen sichergestellt wird, dass ein Zahnarzt rechtzeitig darauf hingewiesen wird, dass ein Wiederaufladen des Akkumulators erforderlich ist.

Hierzu wird zunächst ein erster Schwellwert für den Ladezustand festgelegt, der derart definiert ist, dass bei dessen Unterschreiten ein vollständiger Betrieb des Fußanlassers nur noch für einen bestimmten Zeitraum, bspw. für fünf Arbeitstage, möglich ist. Beim Unterschreiten dieses ersten Schwellwerts kann bspw. mit Hilfe einer LED ein optisches Signal an den Benutzer ausgegeben werden, dass diesem signalisiert, dass ein Laden das Akkumulators erforderlich ist. Auch die Ausgabe eines akustischen Signals, welches bei jedem Betätigen der Tasten bzw. des Pedals des Fußanlassers 10 ausgegeben wird, kann vorgesehen sein, um auf den niedrigen Energiezustand hinzuweisen.

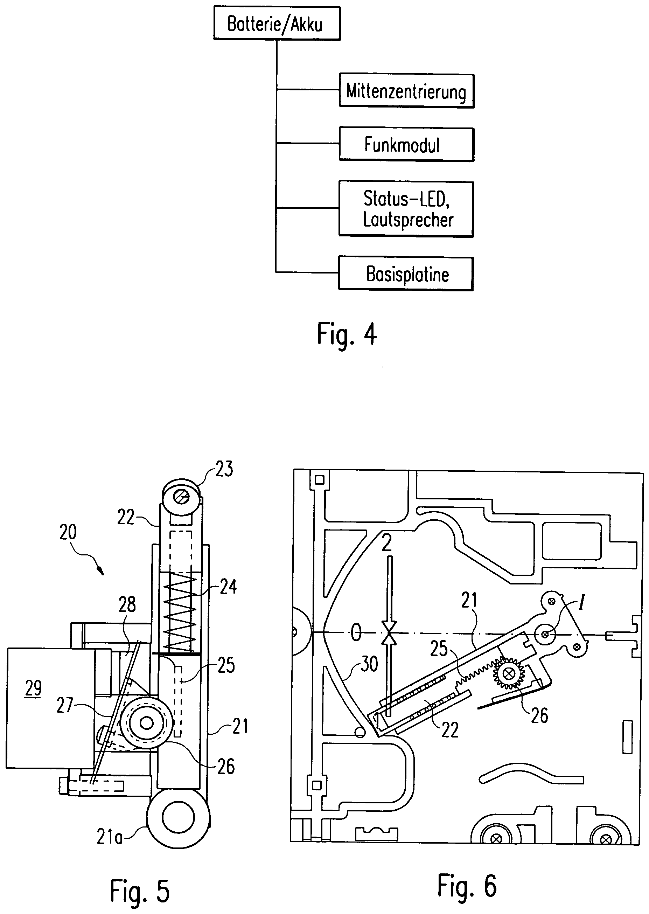

Desweiteren ist ein zweiter Schwellwert festgelegt, bei dessen Unterschreiten ein Notbetrieb eingeleitet wird. Hierbei werden bestimmte Funktionen des Fußanlassers 10 deaktiviert, um weitere Energie zu sparen und nur noch derartige Maßnahmen zur Verfügung zu stellen, welche für eine Aufrechterhaltung der drahtlosen Ansteuerung des Arbeitsplatzes erforderlich sind. Fig. 4 zeigt hierzu schematisch vier verschiedene Komponenten des Fußanlassers 10, welche von der Batterie bzw. dem Akkumulator mit Energie versorgt werden. Eine erste Komponente ist dabei die nachfolgend noch näher beschriebene Mittenzentrierung bzw. deren Umschaltung. Weitere Komponenten sind das Funkmodul, Status-LEDs und ein Lautsprecher zur Information über den Ladezustand bzw. den Betriebszustand des Fußanlassers sowie die eigentliche Steuereinheit, die Basisplatine des Fußanlassers. Diese vier Funktionsblöcke bzw. Komponenten werden von dem Akkumulator gespeist, wobei hierbei auch eine Hierarchie im Hinblick auf die Energieversorgung vorgegeben ist. So benötigt die Mittenzentrierung am meisten Energie, während hingegen die Basisplatine weniger Energie benötigt. Zum Einsparen der Energie bei niedrigem Ladezustand des Akkumulators würde dementsprechend zunächst die Mittenzentrierung, genau genommen die Umschaltung der Mittenzentrierung deaktiviert werden, während hingegen die weiteren Komponenten nach wie vor mit Energie versorgt werden.

Durch die vorbeschriebenen Maßnahmen wird also sichergestellt, dass rechtzeitig ein Wiederaufladen des Akkumulators erfolgt. In jedem Fall wird verhindert, dass durch einen plötzlichen Abfall der Energieversorgung unkontrollierbare Zustände beim Ansteuern des Arbeitsplatzes auftreten.

Abschließend soll die erfindungsgemäße Mittenzentrierung des Pedals 15 des Fußanlassers 10 beschrieben werden. Aufgabe dieser Mittenzentrierung ist es, bei Loslassen des Pedals sicherzustellen, dass dieses in seine Ausgangsposition zurückkehrt. Hierzu ist bspw. aus der DE 33 02 558 A1 der Anmelderin eine Lösung bekannt, bei der die Rückführung auf pneumatischem Wege erfolgt. Da eine derartige Lösung aufgrund der Trennung zwischen Fußanlasser 10 und zentraler Einheit 2 des Arbeitsplatzes 1 allerdings nicht möglich ist, wird nunmehr eine neuartige Lösung vorgeschlagen, die nachfolgend anhand der Figuren 5 bis 8 erläutert werden soll. Fig. 5 zeigt hierbei zunächst die verschiedenen Komponenten der Mittenzentrierung.

Wesentliche Komponenten der Mittenzentrierung 20 sind eine längliche Zylinderführung 21, welche mit ihrem einen Ende 21a um eine Achse I schwenkbar innerhalb des Gehäuses des Fußanlassers gelagert ist. Diese Zylinderführung 21 nimmt einen Kolben 22 auf, der aus drei Elementen besteht. Zum einen aus einem vorderen Endbereich 23, der - wie nachfolgend noch näher beschrieben - entlang einer Führungskurve innerhalb des Fußanlassergehäuses gleitet bzw. abrollt, einer Feder 24, über deren Wirkung eine Rückstellung in die Mittenposition ermöglicht wird, sowie eine Zahnstange 25. Diese Zahnstange 25 wirkt wahlweise mit einer Zahnradaufnahme mit einem Hülsenfreilauf und einem Zahnrad 26 zusammen. Diese Zahnradaufnahme ist an einer Blattfeder 27 gelagert, wobei die Stellung der Blattfeder 27 über einen Excenter 28 eingestellt werden kann, der durch einen Servo 29 angesteuert wird.

Die Fig. 5 und 6 zeigt einen Zustand der verschiedenen Komponenten der Mittenzentrierung 20, in dem diese aktiviert ist. Die Blattfeder 27 ist in dieser Position entspannt, was zur Folge hat, dass sich der Kolben 22 frei innerhalb der Zylinderführung 21 bewegen kann. Das Zahnrad 26 wirkt in diesem Fall nicht mit der Zahnstange 25 des Kolbens zusammen. Wird nun - wie in Fig. 6 dargestellt - das Pedal aus einer Mittenstellung 0 heraus seitlich verschwenkt, so hat dies zur Folge, dass der mit seinem vorderen Ende 23 an einer V-förmigen Führungskurve 30 entlang gleitende Kolben 22 in die Zylinderführung 21 gedrückt wird. Wird das Pedall 5 wieder losgelassen, so bewirkt die Feder 24 ein Herausschieben des Kolbens 23, was zur Folge hat, dass dieser - wiederum entlang gleitend an der V-förmigen Führungskurve 30 - in die Ausgangsposition 0 zurück gestellt wird. Nach einem Loslassen befindet sich dementsprechend das Pedal 15 immer in der Mittenstellung.

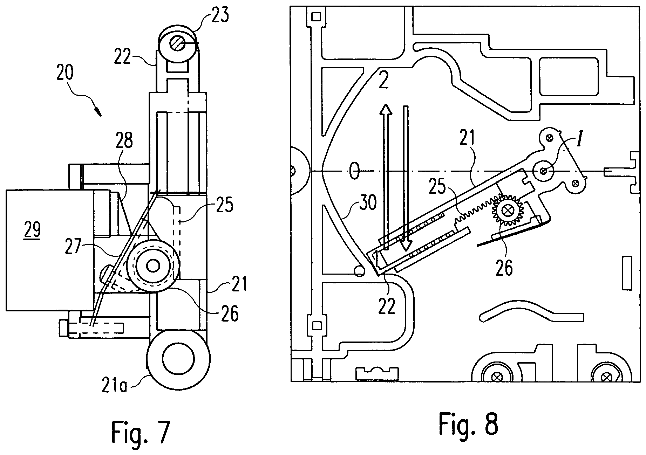

Mit Hilfe des Servos 29 besteht allerdings alternativ dazu auch die Möglichkeit, die Rückstellfunktion zu deaktivieren. In diesem Fall wird der Excenter - wie in Fig. 7 dargestellt - derart angesteuert, dass die Blattfeder 27 gespannt wird, was zur Folge hat, dass das Zahnrad 26 in die Raststange 25 einrastet. Das Zahnrad 26 sitzt wie bereits erwähnt auf einem Freilauf, der die Rückstellung des Kolbens 22 blockiert und den Kolben 22 in dem Zylinder 21 hält. Beim Auslenken in eine der Positionen 1 oder 2 hat dies dann zur Folge, dass keine Rückstellung mehr in die Null-Position erfolgt, da die Federkraft blockiert ist. Das Pedal 15 verharrt dementsprechend in der zuvor eingestellten Position.

Dementsprechend besteht für einen Benutzer jederzeit die Möglichkeit, die Kolbenrückführung wunschgemäß zu aktivieren oder zu deaktivieren. Im aktivierten Zustand wird ein Betrieb erzielt, wie er von herkömmlichen Fußanlassem her bekannt ist. Allerdings besteht auch die Möglichkeit der Deaktivierung, falls dies gewünscht ist. Da allerdings das Umschalten zwischen beiden Betriebszuständen Energie zur Ansteuerung des Servos 29 erfordert, wird bei abfallendem Akkumulator-Ladezustand die Umschaltmöglichkeit unterbunden. In diesem Fall verbleibt dann nur noch eine einzige Betriebsweise.

Durch die dargestellten Maßnahmen wird also ein neuartiger Fußanlasser zur Verfügung gestellt, der eine drahtlose Ansteuerung zahnärztliche Behandlungsplätze ermöglicht. Hierdurch wird der Komfort beim Betrieb und der Durchführung dentaler Behandlungen weiter erhöht.

## Patentansprüche

1. Anordnung zum Ansteuern medizinischer Geräte, insbesondere eines zahnärztlichen Arbeitsplatzes (1), aufweisend:
• eine Bedienvorrichtung (10) zum Generieren von Steuer- und/oder Stellsignalen für das medizinische Gerät,
• eine der Bedienvorrichtung (10) zugeordnete Sendeeinheit zur drahtlosen Übermittlung der Steuer- und/oder Stellsignale sowie
• eine dem medizinischen Gerät zugeordnete Empfangseinheit (18) zum Empfang der Steuer- und/oder Stellsignale sowie zur Weiterleitung dieser an das medizinische Gerät,
wobei die Bedienvorrichtung (10) ein mit einem Fuß zu betätigendes Eingabeelement (15) aufweist, welches schwenkbar gelagert ist,
wobei das schwenkbare Eingabeelement (15) in einer horizontalen Ebene verschwenkbar ist,
wobei die Bedienvorrichtung (10) nicht über ein Kabel mit dem medizinischen Gerät verbunden ist,
**dadurch gekennzeichnet,**
**dass** Mittel (20) vorgesehen sind, über welche das schwenkbare Eingabeelement (15) nach einem Verschwenken aus einer Ausgangsposition automatisch wieder in diese Ausgangsposition zurückgeführt wird,
wobei die Mittel (20) zur Rückführung des schwenkbaren Eingabeelements (15) wahlweise deaktivierbar sind.

2. Anordnung nach Anspruch 1,
**dadurch gekennzeichnet,**
**dass** die Mittel (20) zur Rückführung des schwenkbaren Eingabeelements (15) einen entlang einer Führungskurve gleitenden Kolben (22) umfassen, der in einem schwenkbar gelagerten Führungszylinder (21) aufgenommen ist.

3. Anordnung nach Anspruch 2,
**dadurch gekennzeichnet,**
**dass** die Rückführung des Kolbens (22) und damit des Eingabeelements (15) mittels einer Feder (24) erfolgt.

4. Anordnung nach einem der vorherigen Ansprüche,
**dadurch gekennzeichnet,**
**dass** diese eine interne Energiequelle, insbesondere einen Akkumulator aufweist.

5. Anordnung nach Anspruch 4,
**dadurch gekennzeichnet,**
**dass** Bedienvorrichtung (10) Mittel zur Überwachung des Ladezustands der internen Energiequelle aufweist, welche ein optisches und/oder akustisches Signal abgeben, falls der Ladezustand einen vorgegebenen Grenzwert unterschreitet.

6. Anordnung nach Anspruch 5,
**dadurch gekennzeichnet,**
**dass** ferner bestimmte Komponenten der Anordnung deaktiviert werden, falls der Ladezustand der internen Energiequelle einen weiteren vorgegebenen Grenzwert unterschreitet.

## Claims

1. Arrangement for controlling medical appliances, in particular of a dental work station (1), having:
• an operating device (10) for generating control and/or activation signals for the medical appliance,
• an emitting unit assigned to the operating device (10), for wireless transmission of the control and/or activation signals, and
• a receiving unit (18) assigned to the medical appliance, for receiving the control and/or activation signals and for forwarding these signals to the medical appliance,
wherein the operating device (10) has an input element (15) which is to be actuated by a foot and which is pivotably mounted,
wherein the pivotable input element (15) is pivotable in a horizontal plane,
wherein the operating device (10) is not connected by a cable to the medical appliance,
**characterized in that**
means (20) are provided by which the pivotable input element (15), after pivoting from a starting position, is automatically returned to this starting position,
wherein the means (20) for returning the pivotable input element (15) can optionally be deactivated.

2. Arrangement according to Claim 1, **characterized in that** the means (20) for returning the pivotable input element (15) comprise a piston (22) which slides along a guide curve and which is received in a pivotably mounted guide cylinder (21).

3. Arrangement according to Claim 2, **characterized in that** the return of the piston (22) and thereby of the input element (15) is effected by means of a spring (24) .

4. Arrangement according to one of the preceding claims, **characterized in that** it has an internal energy source, in particular an accumulator.

5. Arrangement according to Claim 4, **characterized in that** the operating device (10) has means for monitoring the state of charge of the internal energy source, which means emit an optical and/or acoustic signal if the state of charge falls below a predefined limit value.

6. Arrangement according to Claim 5, **characterized in that** certain components of the arrangement are moreover deactivated if the state of charge of the internal energy source falls below a further predefined limit value.

## Revendications

1. Arrangement pour commander des appareils médicaux, notamment un poste de travail de dentiste (1), comprenant :
* un dispositif de conduite (10) destiné à générer des signaux de commande et/ou de réglage pour l'appareil médical,
* une unité d'émission, associée au dispositif de conduite (10) et servant à la communication sans fil des signaux de commande et/ou de réglage et
* une unité de réception (18), associée à l'appareil médical et servant à la réception des signaux de commande et/ou de réglage ainsi qu'à la retransmission de ceux-ci à l'appareil médical,
le dispositif de conduite (10) possédant un élément d'entrée (15) à actionner avec un pied et qui est monté pivotant,
l'élément d'entrée (15) pivotant pouvant pivoter dans un plan horizontal,
le dispositif de conduite (10) n'étant pas relié à l'appareil médical par le biais d'un câble,
**caractérisé en ce que** des moyens (20) sont présents, par le biais desquels l'élément d'entrée (15) pivotant est ramené automatiquement dans une position initiale après un pivotement depuis cette position initiale, les moyens (20) de rappel de l'élément d'entrée (15) pivotant pouvant être désactivés au choix.

2. Arrangement selon la revendication 1, **caractérisé en ce que** les moyens (20) de rappel de l'élément d'entrée (15) pivotant comprennent un piston (22) coulissant le long d'une courbe de guidage, lequel est accueilli dans un cylindre de guidage (21) monté pivotant.

3. Arrangement selon la revendication 2, **caractérisé en ce que** le rappel du piston (22) et ainsi de l'élément d'entrée (15) s'effectue au moyen d'un ressort (24).

4. Arrangement selon l'une des revendications précédentes, **caractérisé en ce que** celui-ci possède une source d'énergie interne, notamment un accumulateur.

5. Arrangement selon la revendication 4, **caractérisé en ce que** le dispositif de conduite (10) possède des moyens servant à surveiller l'état de charge de la source d'énergie interne, lesquels délivrent un signal visuel et/ou sonore dans le cas où l'état de charge devient inférieur à une valeur limite prédéfinie.

6. Arrangement selon la revendication 5, **caractérisé en ce que** certains composants de l'arrangement sont en outre désactivés dans le cas où l'état de charge de la source d'énergie interne devient inférieur à une valeur limite prédéfinie supplémentaire.
